# EUROPÄISCHE PATENTSCHRIFT

(11) **EP 0 764 216 B1**
(45) Veröffentlichungstag und Bekanntmachung des Hinweises auf die Patenterteilung: **03.07.2002**
(21) Anmeldenummer: 96914915.2
(22) Anmeldetag: 15.04.1996
(51) Int. Cl.: C12Q 1/26, C12Q 1/00

(54) **VERFAHREN ZUR QUANTITATIVEN BESTIMMUNG VON REVERSIBEL WIRKENDEN INHIBITOREN DER OXIDOREDUKTASEN**
METHOD OF QUANTITATIVE DETERMINATION OF REVERSIBLY ACTING INHIBITORS OF OXIDOREDUCTASES
PROCEDE DE DETERMINATION QUANTITATIVE D'INHIBITEURS D'OXYDOREDUCTASES A EFFET REVERSIBLE

(30) Priorität: 13.04.1995 DE 19514004
(43) Veröffentlichungstag der Anmeldung: 26.03.1997
(73) Patentinhaber: Mischke, Hans-Rudolf, 30459 Hannover (DE)
(72) Erfinder: JÜRGENS, Heike, D-29345 Unterlüss (DE)
(74) Vertreter: Eisenführ, Speiser & Partner
(86) Internationale Anmeldenummer: EP9601573
(87) Internationale Veröffentlichungsnummer: WO9632499

(56) Entgegenhaltungen:
- BE-A- 902 745
- GB-A- 1 605 296
- US-A- 5 356 789
- DATABASE WPI Section Ch, Week 8229 Derwent Publications Ltd., London, GB; Class D16, AN 82-61256e XP002011319 & SU,A,865 904 (AS SIBE PHYS INST) , 25.September 1981
- DATABASE WPI Section Ch, Week 8551 Derwent Publications Ltd., London, GB; Class B04, AN 85-322128 XP002011320 & SU,A,1 160 311 (AS SIBE BIOPHYS INS) , 7.Juni 1985
- SENSORS AND ACTUATORS B, Bd. B24, Nr. 1/03, PART 01, 1.März 1995, Seiten 85-89, XP000521318 CAGNINI A ET AL: "DISPOSABLE RUTHENIZED SCREEN-PRINTED BIOSENSORS FOR PESTICIDES MONITORING"
- Biochem. J. 1972, 127, 321 - 333
- Biochim. Biophys. Acta 1969, 185, 269 - 286
- J. Biol. Chem. 1979, 254, 12104 - 12109

## Beschreibung

In der Lebensmittel-, Pharma- und allgemein chemischen Industrie ist die Erfassung von Meßwerten zur Charakterisierung von Substanzen und als Regelgröße zur Anlagensteuerung von großer Wichtigkeit. Gleiches gilt für die Umwelttechnologie sowie die Wasser-, Abwasser- und Bodenkontrolle.

Herkömmliche selektive Meßverfahren zur Bestimmung solcher Meßwerte und Regelgrößen sind chromatographische Verfahren wie Hochdruckflüssigkeits-Chromatographie (HPLC), Gaschromatographie (GC), naßchemische Verfahren wie maßanalytische Methoden und enzymatische Verfahren unter Verwendung von sogenannten Test-Kits. Nachteilig an dem HPLC-Verfahren ist die lange Analysendauer, die Probenvorbereitung, ferner sind die Verbrauchsmaterialkosten recht hoch, und es ist ein erheblicher instrumenteller Aufwand erforderlich. Das gleiche gilt auch für das GC-Verfahren. Die naßchemischen Verfahren benötigen ebenfalls eine lange Analysendauer, aufwendige Probenvorbereitungen, und es ist bei geringer Selektivität eine große Probemenge erforderlich. Eine lange Analysendauer ist auch für den Einsatz von Test-Kits erforderlich. Ferner ist zu bedenken, daß nur für ausgewählte Analyten Test-Kits im Handel erhältlich sind.

Solche Analyten können beispielsweise sogenannte reversible Inhibitoren sein. Sie umfassen unter anderen Verbindungen wie Carbonsäuren, Aminosäuren, deren Salze, Aldehyde, Amine und Ammoniumsalze. Insbesondere zu nennen wären Essigsäure, Propionsäure, deren Salze, Formaldehyd und Acetaldehyd, N-Methylaminosäuren, Methoxyacetat, 2-Pyrrolcarbonsäure, 2-Thiophencarbonsäure und 2-Furancarbonsäure.

Beispiele für oxidoreduktasen sind Sarkosinoxidase, Monoaminoxidase, L-Lactatmonoxygenase und L-Aminosäureoxidase.

Aus Biochem. J. 1972, 127, 321-333 sind Untersuchungen bekannt, welche die Genetik des stationären Zustands von Enzymen und subzellulären Partikeln betreffende, die mit gebundenen Inhibitoren wechselwirken.

Aus Biochim. Biophys. Acta 1969, 185, 269-286 sind Untersuchungen zur Genetik der reversiblen Inhibierung von enzymkatalysierten Reaktionen durch fest bindende Inhibitoren bekannt.

Aus J. Biol. Chem. 1979, 254, 12104-12109 ist eine vergleichende Untersuchung zur Bewertung von Verfahren zur Abschätzung der Dissoziationskonstante fest bindender Enzyminhibitoren bekannt.

Aus der DE 36 00 563 A1 schließlich ist eine wärmebeständige Sarcosinoxidase N bekannt, die zur oxidativen Zersetzung von Sarcosin eingesetzt werden kann.

Es ist Aufgabe der Erfindung, ein analytisches Verfahren zur Bestimmung solcher Meßwerte und Regelgrößen bereitzustellen, das ohne großen apparativen Aufwand und Aufwand zur Probenaufbereitung in kurzer Zeit verläßliche Ergebnisse zur Konzentration von Analyten liefert. Es ist ferner eine Aufgabe der Erfindung, eine Meßeinrichtung zur Durchführung eines solchen analytischen Verfahrens bereitzustellen.

Gemäß einem ersten Aspekt wird diese Aufgabe durch ein Verfahren zur quantitativen Bestimmung von reversibel wirkenden Inhibitoren enzymatischer Reaktionen gelöst, in dem man ein Enzymsubstrat vorlegt und in Gegenwart des Inhibitors die Enzymaktivität des an der enzymatischen Reaktion beteiligten Enzyms bestimmt und anhand der ermittelten Ergebnisse die Konzentration des reversibel wirkenden Inhibitors berechnet.

In dem erfindungsgemäßen Verfahren ist der Analyt der Inhibitor. Der Inhibitor, der bei konventionellen Verfahren zu einer Meßwertverfälschung führen kann, wird erfindungsgemäß als Meßwert bestimmende Größe benutzt. Die Beeinflussung der Messung durch den Inhibitor wird deshalb zur Messung der Inhibitorkonzentration herangezogen, wobei das Enzymsubstrat nicht, wie in konventionellen Verfahren der Analyt, sondern der konkurrierende Reaktionspartner ist, der zusammen mit dem Inhibitor im Reaktionsansatz vorhanden sein muß. Die Reaktionsteilnehmer, die durch die katalytische Wirkung des Enzyms verbraucht werden oder entstehen, können dann auf herkömmliche Weise zur Detektion herangezogen werden. Die Detektion kann elektrochemisch z.B. amperometrisch oder optisch z.B. photometrisch erfolgen.

Nachfolgend soll die Erfindung beispielhaft an einem Reaktionssystem erläutert werden, das Sarkosinoxidase (SOD) als Enzym und Sarkosin als mögliches Substrat enthält. Als Substrate der Sarkosinoxidase könnten auch N-Methyl-L-leucin, N-Methyl-DL-alanin, N-Methyl-DL-valin, N-Ethylglycin oder heterocyklische Amine eingesetzt werden.

Zur Reaktionsdurchführung kann das Enzym in Lösung oder immobilisiert auf einer Festphase vorliegen.

Dieses Reaktionssystem wird durch die folgende Gleichung dargestellt.

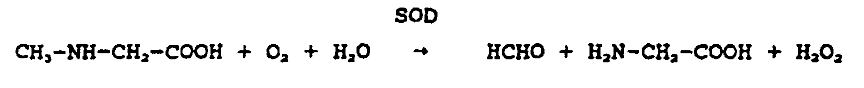

Reversible Inhibitoren in diesem System können beispielsweise Essigsäure, Propionsäure und deren Salze, wie auch Formaldehyd, Acetaldehyd, N-Methylaminosäuren, Methoxyacetat, 2-Pyrrolcarbonsäure, 2-Thiophencarbonsäure und 2-Furancarbonsäure sein. Durch die Zufuhr dieser Analyten in das Reaktionssystem werden der Sauerstoffverbrauch und die Wasserstoffperoxiderzeugung vermindert.

Vorteilhaft ist die hohe Selektivität dieses Reaktionssystems. Durch interferrierende Substanzen hervorgerufene Matrixeffekte können durch Variation von Substrat- und Enzymkonzentration sowie der Pufferzusammensetzung und seiner Konzentration aufgeklärt werden. Des weiteren kann die Selektivität durch die Zudosierung von reversiblen Inhibitoren in das Reaktionssystem beeinflußt werden.

Der Aktivitätsverlust der ansonsten recht stabilen Sarkosinoxidase ist durch Substratkonzentrationserhöhung kompensierbar. Da die inhibierenden Substanzen reversibel wirken, ist keine Regenerierung des Enzyms erforderlich. Die Meßwerterfassung erfolgt ohne weitere Zwischenschritte mit Hilfe einer Sauerstoff- bzw. Wasserstoffperoxiddetektion.

Die zur Durchführung der Bestimmung erforderlichen Reagenzien können in einem sogenannten Test-Kit zusammengefaßt sein und umfassen beispielsweise ein Substrat der Sarkosinoxidase, Sarkosinoxidase, gegebenenfalls in immobilisierter Form, einen Puffer, beispielsweise Phosphatpuffer und Stabilisatoren, je nach Anwendung, wie EDTA.

Eine mögliche Meßeinrichtung zur Durchführung des erfindungsgemäßen Verfahrens umfaßt mindestens zwei Kolbendosierer, die mit einer Reaktionskammer über Leitungen verbunden sind. In den Leitungen sind vorzugsweise Ventile eingebaut, die ein Abstellen, Umleiten oder Zuführen der einzusetzenden Reaktionslösungen ermöglichen. Vorzugsweise können Kolbendosierer für Probe, Puffer und gegebenenfalls weitere Reagenzien eingesetzt werden. Ein Detektor kann entweder in der Reaktionskammer oder aber daran anschließend angeordnet sein, wobei die Art des eingesetzten Detektionssystems grundsätzlich keine Rolle spielt. So kann eine photometrische Detektion erfolgen oder aber beispielsweise eine Bestimmung der Sauerstoffab- bzw. Wasserstoffperoxidzunahme gemessen werden. Soll eine enzymatische Reaktion erfolgen, kann ein Enzym in der Reaktionskammer immobilisiert sein oder aber ebenfalls zur Bestimmung in diese eingebracht werden. Die Durchmischung des Reaktionsgemischs in der Kammer kann durch einen Magnetrührer oder andere Mischsysteme erfolgen. Die Reaktionskammer kann mit einem Abfluß versehen sein. Vorzugsweise werden die Kolbendosierer über einen Motor angetrieben. Kolben und Zylinder der Kolbendosierer bestehen aus Kunststoff, Metall oder Glas.

Zur Durchführung einer Messung werden die Kolbendosierer gleichzeitig oder in zweckmäßiger zeitlicher Folge betätigt, so daß auf der Strecke bis zur Reaktionskammer ein Parallelfluß der Reaktionsteilnehmer eingestellt wird. Nach ausreichender Durchmischung in der Reaktionskammer erfolgt die Messung. Auf diese Weise können diskontinuierliche oder kontinuierliche Abläufe durchgeführt werden.

Das erfindungsgemäße Verfahren wird nachfolgend anhand von Fließinjektions-, Rührkessel- und einem Parallelflußanalysensystem entsprechend der Erfindung dargestellt.

### Fließinjektionssysteme (FIA-Systeme)

Bei der FIA wird ein in das System injiziertes Probenvolumen durch einen kontinuierlich fließenden, nicht segmentierten Träger- bzw. Transportstrom über eine Vermischungs- und Reaktionsstrecke einem Detektor zugeführt. Dabei verbreitert sich die injizierte Probenzone (Dispersion) und reagiert mit den Bestandteilen des Trägerstroms. Die durch den chemischen Umsatz bedingten Konzentrationsänderungen der beteiligten Komponenten führen am Detektor zu Meßwertänderungen, die charakteristisch für die Konzentration der zu bestimmenden Substanz in der injizierten Probe sind.

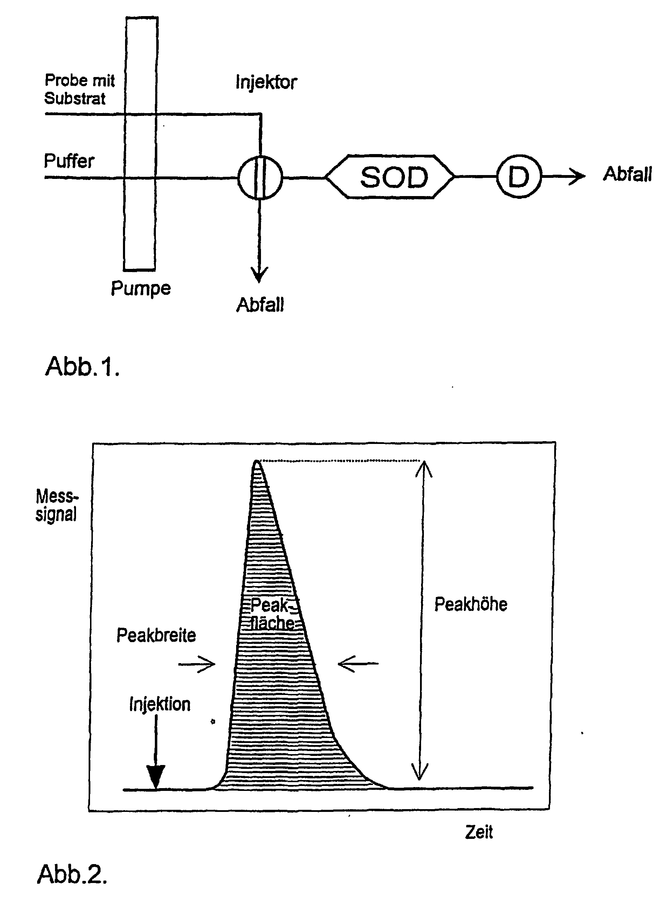

In der Abbildung 1 wird der Analyt (Essigsäure) zusammen mit dem Substrat (Sarkosin) als Probe in den als Trägerstrom eingesetzten Betriebspuffer injiziert. Die Vermischungs- und Reaktionsstrecke enthält das immobilisierte Enzym Sarkosinoxidase, das mit dem Analyten und dem Substrat der Probe reagiert. Die Sauerstoffabnahme bzw. Wasserstoffperoxidzunahme wird mit Hilfe einer Sauerstoff- bzw. Wasserstoffperoxidelektrode gemessen.

Ein typisches Meßsignal ist in der Abbildung 2 dargestellt. Die Peakhöhe H oder Fläche A ist proportional der Probenkonzentration.

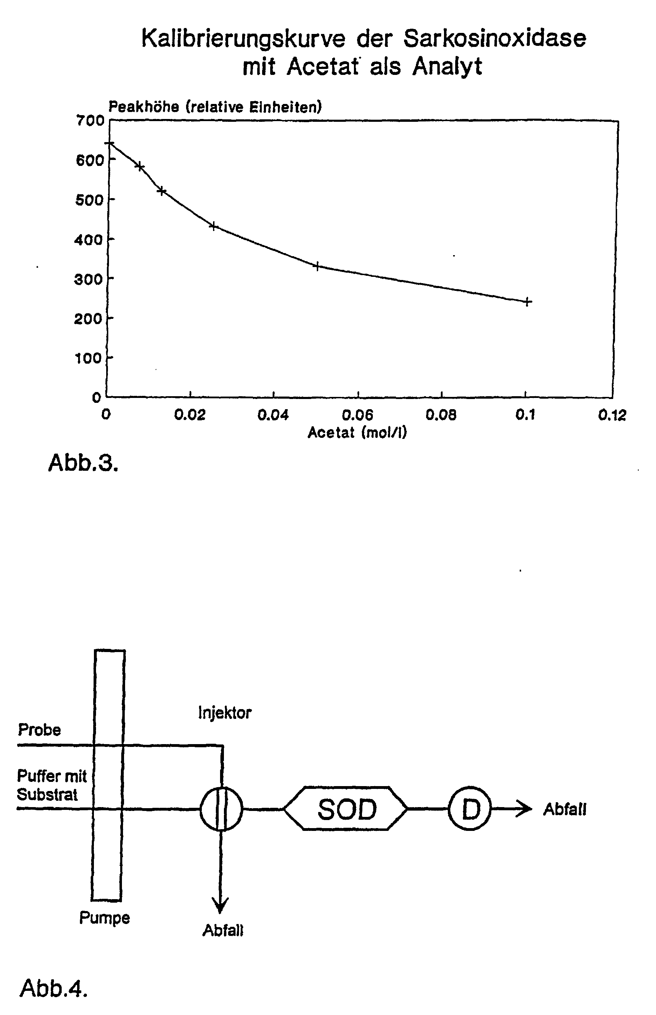

Bei der Kalibrierung über die Peakhöhen werden Proben verschiedener Analytkonzentrationen injiziert und die dazugehörigen Peakhöhen in Abhängigkeit von der Analytkonzentration wie in Abbildung 3 aufgetragen.
Die zu bestimmende Probe mit unbekannter Analytkonzentration wird auf gleiche Weise injiziert und anhand der Peakhöhe mit Hilfe der Kalibrierungskurve quantitativ bestimmt.

Infolge der Trennung von Enzym und Reaktionsmedium verbleibt das immobilisierte Enzym unter Erhalt der katalytischen Wirksamkeit im System, während ständig neue Endukte zugeführt bzw. entstandene Produkte abgeführt werden.

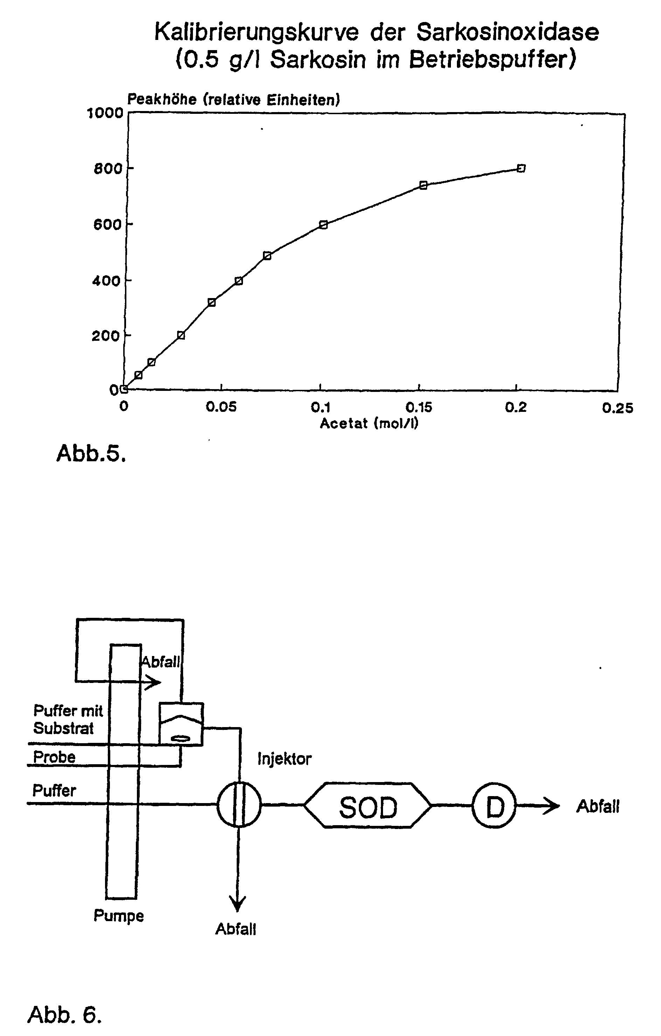

In der Abbildung 4 wird das Substrat in den Pufferstrom gegeben und die Kalibrierung durch Injektion des Analyten vorgenommen. Die Analyse der unbekannten Probe kann wiederum anhand der Kalibrierungskurve, wie sie in Abbildung 5 dargestellt ist, über die Peakhöhen erfolgen.

Die Abbildung 6 zeigt die automatische Zufuhr des Analyten sowie des Substrats über eine Vermischungseinheit.

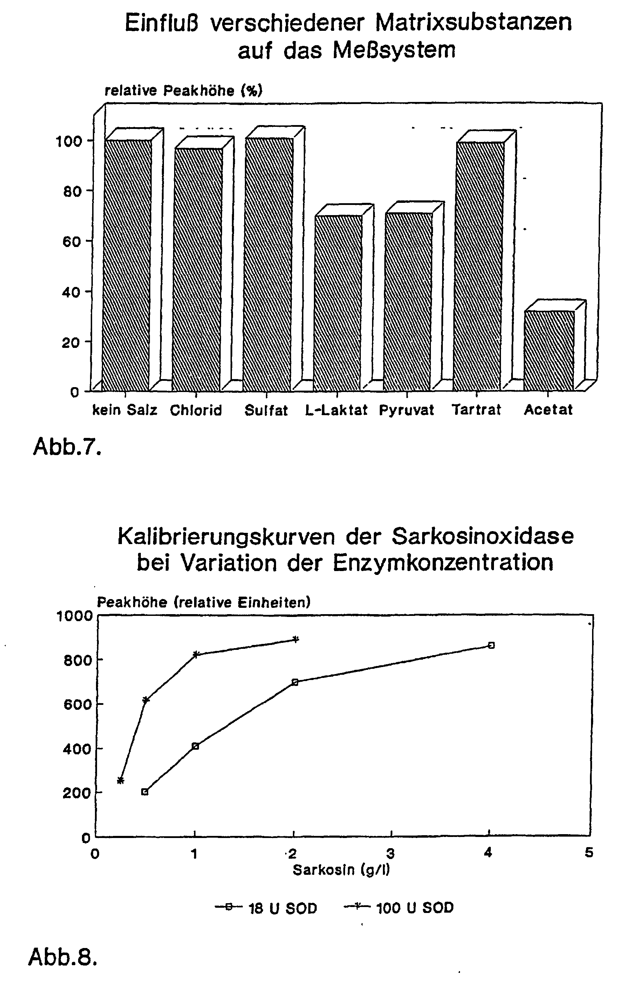

In Abbildung 7 ist die unterschiedliche Inhibierungsstärke von Substanzen, die häufig in biotechnologischen Medien auftreten, dargestellt. Es wird eine in derartigen Medien maximale Konzentration von 0,1 mol/l gewählt. Es ist zu erkennen, daß das Meßsystem besonders stark durch Acetat inhibiert wird, aber nur geringfügig durch die anderen Substanzen.

Abbildung 8 zeigt zwei Kurvenverläufe verschiedener Sarkosinoxidaseaktivitäten, wobei 18 U und 100 U Sarkosinoxidase bei steigender Substratkonzentration eingesetzt werden. Es ist zu erkennen, daß die Kurvenverläufe unterschiedliche lineare Meßbereiche aufweisen. Bei der Sarkosinoxidaseaktivität von 18 U erstreckt sich der lineare Meßbereich bis 2 g/l bei geringer Meßempfindlichkeit, während sich bei der Aktivität von 100 U ein linearer Meßbereich bis 0,5 g/l Sarkosin bei hoher Meßempfindlichkeit ergibt.

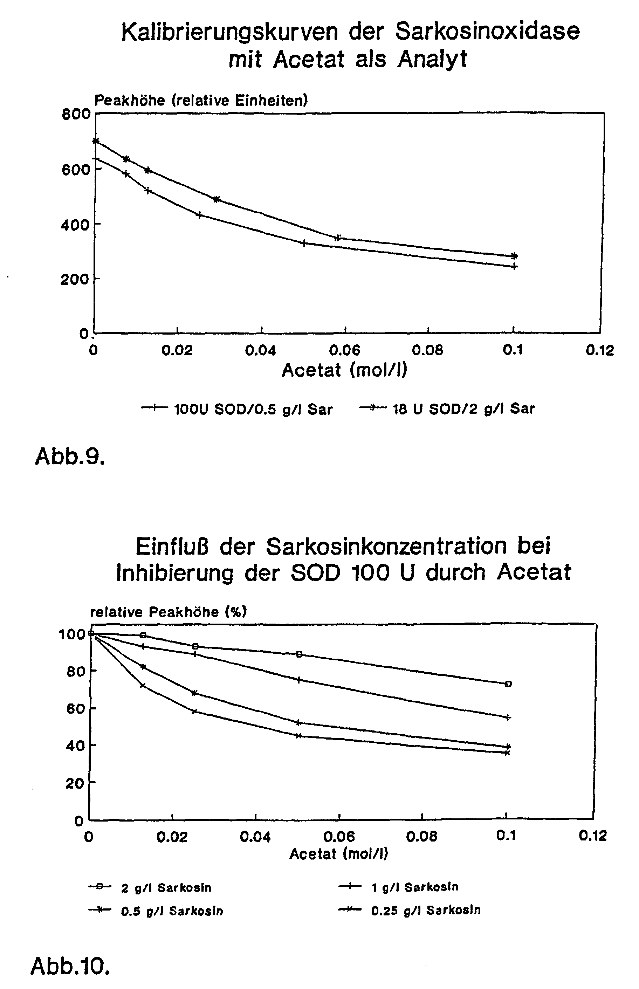

Die zu den unterschiedlichen Enzymaktivitäten gehörenden Kalibrierungskurven mit Acetat als Analyt sind in Abb. 9 dargestellt. Die Substratkonzentration beträgt bei der Sarkosinoxidaseaktivität von 100 U 0,5 g/l Sarkosin, während bei der geringeren Sarkosinoxidaseaktivität 2 g/l Sarkosin eingesetzt werden muß, um nahezu die gleichen Peakhöhen zu erreichen. Die Messungen zeigen, daß eine eventuelle Abnahme der Aktivität des Enzyms durch eine Zunahme der Substratkonzentration unter Aufrechterhaltung des Kurvenverlaufs ausgeglichen werden kann. Vorraussetzung dafür ist, daß bei beiden Enzymaktivitäten Substratkonzentrationen gewählt werden, die in den in Abbildung 8 dargestellten linearen Meßbereichen liegen.

Abbildung 10 zeigt den Einfluß von vier unterschiedlichen Sarkosinkonzentrationen auf die Inhibierung der Sarkosinoxidaseaktivität (100 U) in Abhängigkeit von der Acetatkonzentration. Es ist zu erkennen, daß bei 0,25 g/l bzw. 0,5 g/l Sarkosin eine erhebliche Inhibierung erfolgt, wobei sich besonders bei geringen Acetatkonzentrationen bis zu 0,02 mol/l eine hohe Meßempfindlichkeit einstellt. Demgegenüber ist bei einer Konzentration von 1 g/l bzw. 2 g/l Sarkosin nur eine geringe Inhibierung zu beobachten, welche aber einen großen Bereich der Acetatkonzentration nahezu linear abdeckt.

Die Messungen zeigen, daß durch Wahl der Substratkonzentration die Stärke der Inhibierung variiert werden kann, so daß sich ein flexibles Meßsystem ergibt, das im Hinblick auf das Meßproblem optimal eingestellt werden kann.

### Rührkesselsystem

Im Gegensatz zum FIA-System arbeitet das Rührkesselsystem diskontinuierlich. Zur Durchführung einer Messung werden alle erforderlichen Reaktionsteilnehmer in die Reaktionskammer des Rührkesselsystems eingebracht und der Reaktionsverlauf mit geeigneten Detektoren über eine bestimmte Zeitdauer ermittelt. Der Reaktionsansatz wird anschließend komplett verworfen, bevor ein neuer Meßzyklus beginnt.

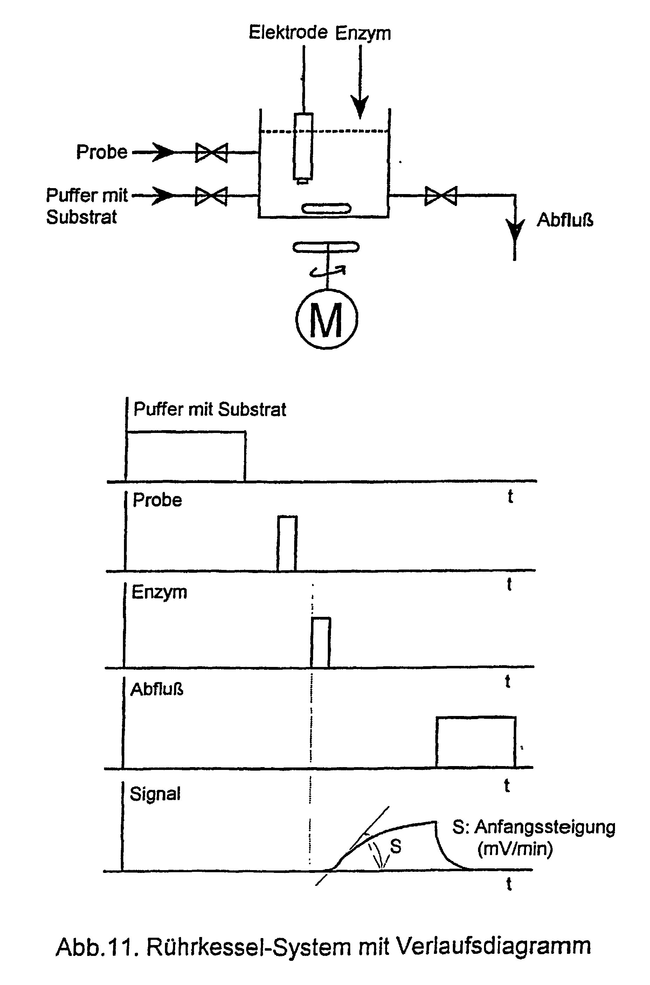

In dem Rührkesselsystem gemäß Abbildung 11 besteht der Reaktionsansatz aus Probe, Substrat, Betriebspuffer und Enzym, wobei die Probe und das Enzym nacheinander zugegeben werden und die Probe den Analyten wie Essigsäure in unterschiedlichen Konzentrationen enthält. Die Sauerstoffabnahme oder die Wasserstoffperoxidzunahme wird mit Hilfe einer Sauerstoff- oder Wasserstoffperoxidelektrode als zeitabhängige Größe gemessen werden, wobei sich das Meßergebnis aus der Anfangssteigung des Signals ergibt.

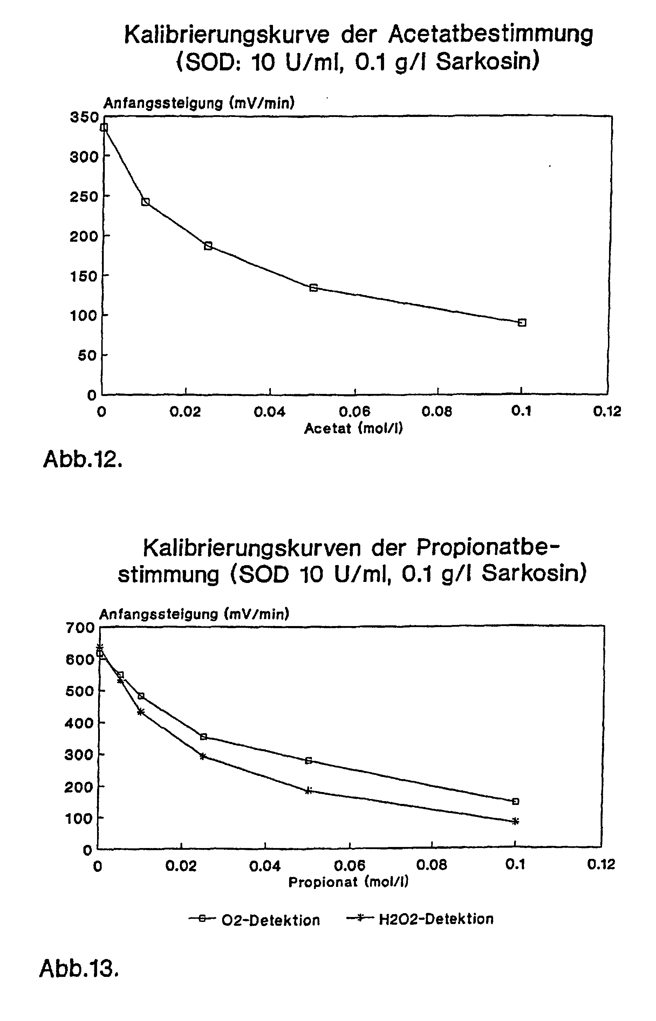

Bei der Kalibrierung über die Anfangssteigung werden Proben verschiedener Analytkonzentration zugegeben und die dazugehörigen Anfangssteigungen in Abhängigkeit von der Analytkonzentration aufgetragen (siehe Abbildungen 12, 13 und 14). Die zu bestimmende Probe mit unbekannter Analytkonzentration wird auf gleiche Weise zum Reaktionsansatz gegeben und anhand der Anfangssteigung mit Hilfe der jeweiligen Kalibrierungskurve analysiert.

In der Abbildung 13 sind die Kalibrierungskurven für die Propionatmessung bei gleichzeitiger Sauerstoff- und Wasserstoffperoxiddetektion gezeigt.

In Abbildung 15 sind die relativen Anfangssteigungen in Abhängigkeit der jeweiligen Analytkonzentration dargestellt. Es ist ersichtlich, daß sich bei Acetat und Propionat ähnliche Verläufe ergeben, während bei Acetaldehyd eine geringere und bei Formaldehyd eine wesentlich stärkere Inhibierung der Sarkosinoxidase erfolgt. Die Meßempfindlichkeit ist bei den Analyten im niedrigen Konzentrationsbereich am höchsten.

Abbildung 16 zeigt den Einfluß der Inhibitoren Acetat, Propionat und Acetaldehyd und Formaldehyd auf die Aktivität der Sarkosinoxidase. Es ist zu erkennen, daß Acetat und Propionat bei 0,1 g/l Sarkosin eine wesentlich größere Inhibierung der Sarkosinoxidase bewirken als bei 1,0 g/l Sarkosin, während die Inhibierung durch Acetaldehyd und Formaldehyd nur geringfügig durch die Substratkonzentration beeinflußt wird. Die Messungen zeigen, daß auch beim Rührkesselsystem durch Variation der Substratkonzentration die Stärke der Inhibierung und somit die Selektivität des Reaktionssystems beeinflußt werden kann.

### Parallelflußanalysensystem (PFA-System)

Abbildung 17 zeigt eine erfindungsgemäße Meßvorrichtung mit drei Kolbendosierern, die jeweils den Puffer, das Substrat und die den Analyten enthaltende Probe oder gegebenenfalls eine von mehreren möglichen Kalibrierungslösungen (St) fördern. Die Kolbendosierer sind mit einer Reaktionskammer über Leitungen verbunden, die jeweils mit einem Ventil ausgerüstet sind. Die Reaktionskammer enthält eine immobilisierte Sarkosinoxidase. Der in der Reaktionskammer angeordnete Detektor ist eine Sauerstoff- bzw. Wasserstoffperoxidelektrode.

Die Steuerung der drei Kolbendosierer erfolgt derart, daß die mit Substrat und Puffer durchmischte Probe in der Reaktionskammer zum Stoppen gebracht wird. Die Sauerstoffabnahme bzw. Wasserstoffperoxidzunahme wird als zeitabhängige Größe gemessen, wobei sich wie im Rührkesselsystem das Meßergebnis aus der Anfangssteigung des Meßsignals ergibt.

Zur Kalibrierung über die Anfangssteigungen werden Proben verschiedener Analytkonzentration zudosiert und die dazugehörigen Anfangssteigungen in Abhängigkeit von der Analytkonzentration aufgetragen. Die zu bestimmende Probe mit unbekannter Analytkonzentration wird auf gleiche Weise in die Reaktionskammer eingebracht und gemessen.

Eine Variation der Mischungsverhältnisse von Puffer, Substrat und Probe ist mit Hilfe des PFA-Systems ohne apparative Änderungen durch Veränderung von Dosiergeschwindigkeit und Ansaugvolumen der jeweiligen Kolbendosierer durchführbar. Dies ist besonders bei der Untersuchung von Proben unterschiedlicher Matrix erforderlich. Weiterhin kann auf eine externe Probenverdünnung als Probenvorbereitung verzichtet werden.

## Patentansprüche

1. Verwendung von Essigsäure, Propionsäure und/oder deren Salzen, Formaldehyd, Acetaldehyd, eine N-Methylaminosäure, Methoxyacetat, 2-Pyrrolcarbonsäure, 2-Thiophencarbonsäure oder 2-Furancarbonsäure als reversibel wirkender Inhibitor von Sarkosinoxidasen.

2. Verfahren zur quantitativen Bestimmung der Konzentration eines reversibel wirkenden Inhibitors der Oxidoreduktasen,
wobei der Inhibitor ausgewählt ist aus der Gruppe, die aus Essigsäure, Propionsäure und deren Salzen, Formaldehyd, Acetaldehyd, N-Methylaminosäure, Methoxyacetat, 2-Pyrrolcarbonsäure, 2-Thiophencarbonsäure und 2-Furancarbonsäure besteht,
wobei als Enzymsubstrat Sarkosin, N-Methy-L-leucin, N-Methyl-DL-alanin, N-Methyl-DL-valin, N-Ethylglycin oder ein heterocyclisches Amin vorgelegt wird,
und wobei in Gegenwart des Inhibitors die Enzymaktivität von Sarkosinoxidase gemessen wird, welche mit dem Enzymsubstrat zusammengebracht wurde.

## Claims

1. The use of acetic acid, propionic acid and/or their salts, formaldehyde, acetaldehyde, an N-methylamino acid, methoxyacetate, pyrrole-2-carboxylic acid, thiophene-2-carboxylic acid or 2-furancarboxylic acid as a reversibly acting inhibitor of sarcosine oxidases.

2. A process for the quantitive determination of the concentration of a reversibly acting inhibitor of oxidoreductases,
the inhibitor being selected from the group comprising acetic acid, propionic acid and their salts, formaldehyde, acetaldehyde, N-methylamino acid, methoxyacetate, pyrrole-2-carboxylic acid, thiophene-2-carboxylic acid and 2-furancarboxylic acid,
sarcosine, N-methyl-L-leucine, N-methyl-DL-alanine, N-methyl-DL-valine, N-ethylglycerine or a heterocyclic amine being provided as the enzyme substrate,
and the enzyme activity of sarcosine oxidase, which was brought into contact with the enzyme substrate, being measured in the presence of the inhibitor.

## Revendications

1. Utilisation de l'acide acétique, de l'acide propionique et/ou de leurs sels, du formaldéhyde, de l'acétaldéhyde, d'un N-méthyl aminoacide, d'un méthoxyacétate, de l'acide 2-pyrrol-carboxylique, de l'acide 2-thiophène-carboxylique ou de l'acide 2-furane carboxylique en tant qu'inhibiteur à effet réversible de l'oxydase de sarcosine.

2. Procédé de détermination quàntitative de la concentration en inhibiteur d'oxydoréductase à effet réversible,
l'inhibiteur étant choisi dans le groupe constitué de l'acide acétique, l'acide propionique et leurs sels, le formaldéhyde, l'acétaldéhyde, un N-méthyl aminoacide, un méthoxyacétate, l'acide 2-pyrrol-carboxylique, l'acide 2-thiophène-carboxylique et l'acide 2-furane carboxylique
la sarcosine, la N-méthyl-L-leucine, la N-méthyl-DL-alanine, la N-méthyl-DL-valine, la N-éthylglycine ou une amine hétérocyclique servant de substrat enzymatique,
et l'activité enzymatique de l'oxydase de sarcosine, après mélange avec le substrat enzymatique, étant mesurée en présence de l'inhibiteur.
